(19) Europäisches Patentamt
European Patent Office
Office européen des brevets

(11) Publication number : **0 491 538 A1**

(12) **EUROPEAN PATENT APPLICATION**

(21) Application number : **91311659.6**

(22) Date of filing : **16.12.91**

(51) Int. Cl.$^5$ : **C07D 307/33,** C07D 263/52, C07C 237/22

(30) Priority : **17.12.90 US 628045**

(43) Date of publication of application : **24.06.92 Bulletin 92/26**

(84) Designated Contracting States : **CH DE FR GB IT LI NL**

(71) Applicant : **MERCK & CO. INC.**
**126, East Lincoln Avenue P.O. Box 2000**
**Rahway New Jersey 07065-0900 (US)**

(72) Inventor : **Decamp, Ann E.**
**136 Sanford Avenue**
**North Plainfield, NJ 07060 (US)**
Inventor : **Shinkai, Ichiro**
**1101 Prospect Street**
**Westfield, NJ 07090 (US)**
Inventor : **Kawaguchi, Alan T.**
**486 West Inman Avenue**
**Rahway, NJ 07065 (US)**
Inventor : **Volante, Ralph P.**
**22 Hawthorne Lane**
**East Windsor, NJ 08520 (US)**

(74) Representative : **Thompson, John Dr. et al**
**Merck & Co., Inc. European Patent Department Terlings Park Eastwick Road**
**Harlow, Essex CM20 2QR (GB)**

(54) **Stereocontrolled production of hydroxyester, hydroxyamide, and lactone compounds from chiral alpha-amino aldehydes.**

(57) A process for stereocontrolled preparation of a medicinally significant hydroxyester, hydroxyamide or lactone compound of structural formula :

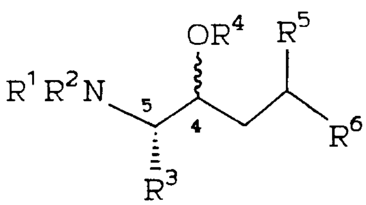

comprises the addition of homoenolate equivalents to chiral α-amino aldehydes. The hydroxyester, hydroxyamide, or lactone reaction products are useful as inhibitors of the HIV protease or of renin, or as intermediates in the preparation of inhibitors of the HIV protease or renin.

EP 0 491 538 A1

## BACKGROUND OF THE INVENTION

Diastereoselective homologation of protected α-amino aldehydes to give 4S,5S-hydroxyester products has been a long sought after goal in the area of peptide chemistry [Jurczak, J., and Golbiowski, A., Chem. Rev., 89, 149 (1989)]. These hydroxyesters are intermediates in the synthesis of medicinally important enzyme (renin and HIV protease) inhibitors which incorporate the hydroxyethylene dipeptide isostere unit [Melnick, M. J., et al., Tetrahedron Lett., 31, 961, and references therein (1990); Prasad, J. V. N. V., and Rich, D. H., Tetrahedron Lett., 31, 1803 (1990); Bradbury, R. H., et al., Tetrahedron Lett. 30, 3845 (1989); Chakravarty, P. K., et al., Tetrahedron Lett., 30, 415 (1989); Kempf, D. J., J. Org. Chem., 51, 3921 (1986); Fray, A. H., et al., J. Org. Chem., 51, 4828 (1986); Evans, B. E., et al., J. Org. Chem., 50, 4615 (1985); Klutchko, S., et al., Synthetic Commun., 19, 2573, and references therein (1989); Andrew, R. G., et al., Tetrahedron Lett., 28, 6535 (1987); Mikami, K., et al., Tetrahedron Lett., 31, 3909 (1990); Nishi, T., et al., Tetrahedron Lett., 29, 6327 (1988); Takemoto, Y., et al., Tetrahedron Lett., 31, 217 (1990)]. Many synthetic approaches have been explored for efficient and stereocontrolled preparation of these 4S,5S-hydroxyesters and their corresponding lactones. However, a one-step homologation to the desired hydroxyester oxidation state has not been reported. Typically, control of the 4S-stereocenter has been problematic.

This invention provides a direct, high yielding, stereoselective route to lactone, hydroxyamide and hydroxyester compounds via metallo-homoenolate methodology [Fukuzawa, S., et al., J. Org. Chem., 55, 1628 (1990) and references therein; Verlhac, J., and Pepeyre, M., Tetrahedron, 46, 6399 (1990); Furstner, A., Synthesis, 571, (1989); Nakamura, E., et al., J. Am. Chem. Soc., 109, 8056 (1987); Nakamura, E., et al., J. Am. Chem. Soc., 108, 3745 (1986); Nakamura, E., et al., Tetrahedron Lett., 28, 337 (1987)].

A significant utility of this process is the preparation of diastereomeric compounds having the ability to inhibit certain proteolytic enzymes, including renin [U.S. Patent 4,661,473; Evans, B. E. et al., J. Org. Chem., 50, 4615 (1985); Evans, B. E. et al., "A Stereocontrolled Synthesis of Hydroxyethylene Dipeptide Isosteres," Proc. Am. Pep. Symp., 9, 743 (1985)], and the protease of Human Immunodeficiency Virus (HIV). The HIV protease has been recognized as a possible target for therapeutics aimed at prevention or treatment of infection by HIV, as interruption of processing of viral structural proteins by the protease prevents production of infectious virus [Crawford, S. et al., J. Virol. 53. 899 (1985); Toh, H. et al., EMBO J. 4, 1267 (1985); Power, M. D. et al., Science 231, 1567 (1986); Pearl, L. H. et al., Nature 329, 351 (1987). See also U.S.S.N. 236,495, 328,645, 235,841, 328,644, 180,507, 236,084, 328,643, 597,286, 595,913, corresponding to EP-A-0356223, EP-A-0357332, EP-A-0337714, EP-A-0434365 and copending European patent application no. 91309302.7].

## SUMMARY OF THE INVENTION

The novel process of the invention is summarized according to SCHEME I:

SCHEME I

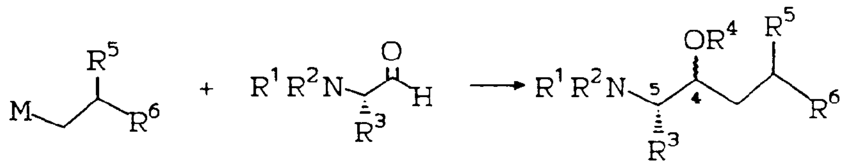

I II III

whereby a metallo-homoenolate of structural formula I is reacted with an α-amino aldehyde of structural formula II to yield a hydroxyester, hydroxyamide, or lactone. The reaction may be run in a single step and may be adapted to favor production of predominantly 4S or 4R diastereomeric product having a structural formula III. Compounds comprehended by III are useful in the preparation of inhibitors of proteolytic enzymes, especially of the HIV protease, and of renin, and some of the compounds of formula III are themselves useful inhibitors of the HIV protease.

## DETAILED DESCRIPTION OF THE INVENTION

This invention provides a direct, high yielding, stereoselective route to lactone, hydroxyamide or hydroxyester compounds via metallo-homoenolate methodology. The process is represented in Scheme I as the addition of the metallo-homoenolate, I, to a chiral α-amino aldehyde, II, to yield the hydroxyester, hydroxyamide or lactone, III, wherein the ratio of 4S,5S:4R,5S product is controlled by the nature of the aldehyde's α-amino protecting groups, $R^1$ and $R^2$, and by the nature of the metallo-homoenolate, I:

SCHEME I

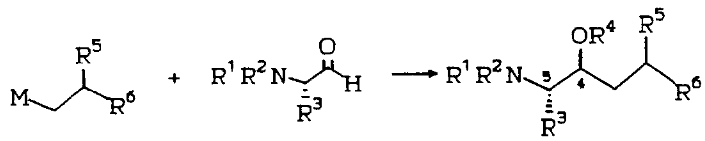

I II III

at a temperature between about -50°C and ambient temperature in a solvent selected from $CH_2Cl_2$, toluene, methyl-tert-butyl ether, diethyl ether, hexane, and $CCl_4$ or any combination of these, wherein:

$R^1$ and $R^2$ are independently:
- a) hydrogen with the proviso that $R^1$, and $R^2$ may not both be H,
- b) t-Boc, Aoc, Adc, Mcb, Mch, Cbz, Alloc, or Fmoc,
- c) benzyl,
- d) lower alkyl,
- e) $CH_3O(CH_2CH_2O)_nCO$-, wherein n is from 1 to 10,
- f) tetrahydrofuranyloxy-CO-, or
- g) R-CO-, wherein R is an amino acid or peptide of up to five amino acids;

$R^3$ is:
- a) benzyl,
- b) cyclohexyl-$CH_2$-,
- c) isobutyl,
- d) benzyl-O-benzyl-, or
- e) cinnamyl-;

$R^4$ is:
- a) hydrogen,
- b) a bond to $R^6$, when $R^6$ is carbonyl, or
- c) (lower alkyl)$_3$Si-, t-butyl(lower alkyl)$_2$Si;

$R^5$ is:
- a) hydrogen,
- b) lower alkyl,
- c) benzyl,
- d) -$CH_2$-phenyl-O-$R^9$, or
- e) -$CH_2$-CH=CH-phenyl-O-$R^9$;

$R^9$ is:
- a) benzyl,
- b) -$CH_2CH_2$-O-$R^{14}$, or
- c)

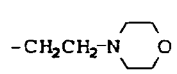

;

$R^6$ is:

a) $-COOR^7$,

b) -CO-, when bonded to $R^4$ to form a lactone,

c)

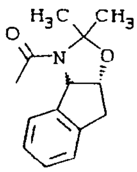

d)

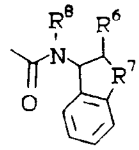

wherein:

$R^{12}$ is -H, or -OH;

$R^{13}$ is $-CH_2-O-$,

or 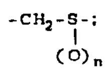

n = 0, 1, or 2;

e)

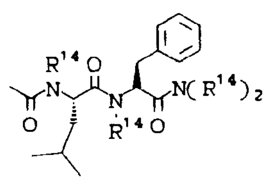

f)

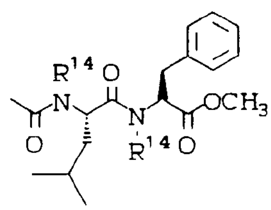

g)

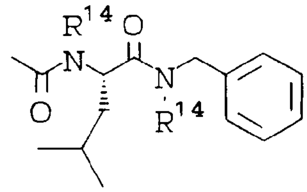

h)

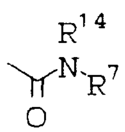

i)

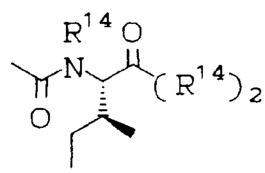

j)

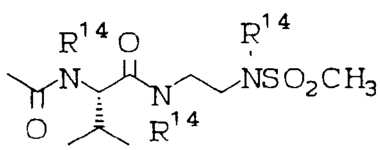

$R^7$ is:

a) lower alkyl, or
b) benzyl;

$R^{14}$ is a removable protecting group selected from among:

a) t-Boc, Aoc, Adc, Mcb, Mch, Cbz, Alloc, or Fmoc, or
b) benzyl;

M is:

a) $TiR^8$,
b) $Sn(R^{10})_3$
c) $ZnR^{11}$,
d) $SmR^{11}$, or
e) $Ce(R^{11})_2$;

$R^8$ is:

a) $Cl_x(OiPr)_{3-x}$, or
b) $Br_x(OiPr)_{3-x}$;

x is an integer from 0 to 3;
$R^{10}$ is -lower alkyl, and
$R^{11}$ is I, Br, or Cl.

It is to be understood that the nitrogen and oxygen protecting groups provided for in the definitions above may be removed to generate free amino and hydroxyl compounds. Thus, the $R^{14}$ group, wherever it appears, may be removed according to methods known in the art. Furthermore, the cyclic-aminal protecting acetonide (also called isopropylidene), present in $R^6$ (c) may similarly be removed.

As used herein, lower alkyl means straight or branched chain alkyls of one to five carbon atoms. When any one variable occurs more than one time in a molecule, its definition on each occurrence is independent of its definition at every other occurrence. Ph stands for phenyl, Et for ethyl, Aoc, t-Boc, etc. are amino or urethane protecting groups known in the art, (t-Boc is preferred as it is easily removed under mild conditions). PhMe is toluene, AcOH is acetic acid, and OiPr is isopropoxy.

In a preferred embodiment of the novel process, the metal used is titanium, as shown in Scheme II below. The titanium homoenolate, IV, is prepared, for example, from commercially available ethyl-3-bromopropionate, V, by modification of the procedure of Yoshida, et al., [Ochiai, H., et al., J. Org. Chem. 53, 1343 (1988); Tamaru, Y., et al., Org. Syn. 67, 98 (1988)]. The bromide is converted to ethyl-3-iodopropionate, VI, by reaction with sodium iodide. The iodide is metallated with zinc-copper couple [Smith, R. D., and Simmons, H. E., Org. Syn., Collect. Vol 5, 855 (1973)] in toluene for one hour at ambient temperature followed by 3 hours at 80°C in the presence of N,N-dimethylacetamide to give the iodozinc-homoenolate species, VII. The reactive homoenolate anion, IV, is generated by transmetallation of the iodozinc-homoenolate with one of several chlorotitanium isopropoxide species by modification of the procedure of Reetz, M. T., et al., [Angew. Chem. Suppl., 1899 (1982)]:

SCHEME II

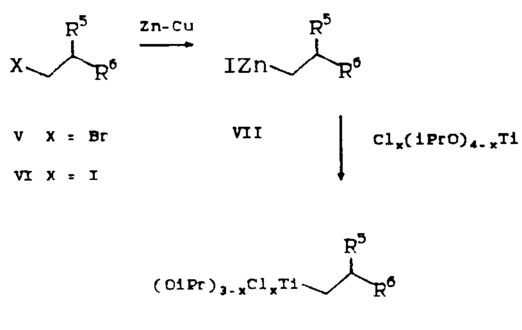


IV

Reaction of IV of Scheme II with II of Scheme I provides III as a mixture of diastereomers, which may be lactonized by treatment with acid in an organic solvent, preferably acetic acid in toluene.

The stereoselectivity of the homoenolate addition is very dependent on which titanium species is used for the transmetallation step. The 4S,5S diastereoselectivity of the reaction increased as the number of chlorines on the titanium homoenolate anion increased [Reetz, M. T., et al., Angew. Chem., Int. Ed. Engl. 26, 1141 (1987)]. For example, the ratio 4S,5S : 4R,5S was 2.8:1, 9:1 and 16:1 over the series of homoenolates VIII, IX, and X. However, the ratio was 3.6:1 and the yield was down to 18% where XI, prepared by this method, was used as the homoenolate. Thus, for enhancement of 4S,5S:4R,5S ratio, x in Scheme II above is preferably 2. The results for homoenolate XI prepared without the zinc homoenolate intermediate by the method of Nakamura and Kuwajima [J. Am. Chem. Soc., 108, 3745 (1986)] produced the hydroxyesters in a 1:5 ratio and a 32% yield:

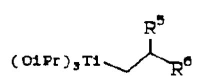
VIII

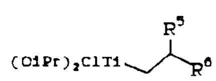
IX

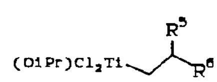
X

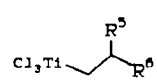
XI

In a preferred embodiment of this process, shown below in Scheme III, a t-Boc protected aldehyde XII is reacted with a dichloroisopropoxytitanium homoenolate XIII, following which the hydroxyester compound XIV may be lactonized to XV:

SCHEME III

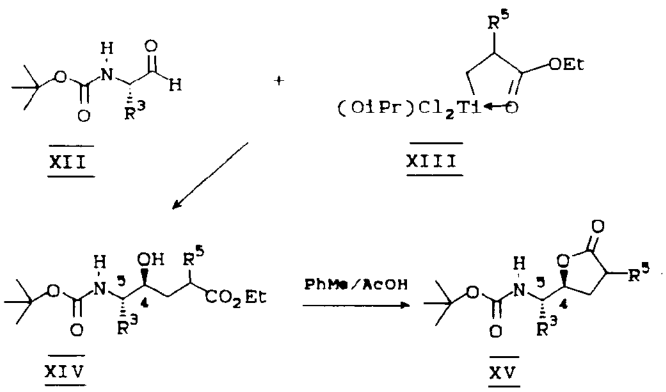


XII XIII

XIV XV

The hydroxyester XIV and lactone XV are useful intermediates in the preparation of HIV protease inhibitory compounds (See US Application Serial No's. 236,495, 328,645, 235,841, 328,644, 180,507, 236,084, 328,643, 597,286, 595,913; corresponding to EP-A-0356223, EP-A-0357332, EP-A-0337714, EP-A-0434365 and copending European patent application no. 91309302.7 ).

The use of two equivalents of homoenolate I anion relative to aldehyde II results in the highest diastereoselectivity. The ratio of isomers decreased from 9:1 to 6:1 as the stoichiometry of homoenolate anion was increased from two to six equivalents [Kiyooka, S., et al., J. Org. Chem. 54, 5409 (1989)]. Fewer than two equivalents resulted in incomplete consumption of aldehyde. Thus, a preferred ratio of homoenolate to aldehyde is between about 6:1 and 1:1 and is most preferably about 2:1.

Reetz has shown that the nitrogen protecting group has an influence on the diastereoselectivity of nucleophilic additions to α-amino aldehydes [Reetz, M. T., et al., Angew. Chem. Int. Ed. Engl. 26, 1141 (1987); Reetz, M. T., et al., Tetrahedron : Asymmetry, 1, 375 (1990)]. In another preferred embodiment of this invention, a N,N-dibenzyl α-amino aldehyde XVI, SCHEME IV, is reacted with the homoenolate anion X, vastly favoring formation of the non-chelation controlled product XVII (4R,5S:4S,5S > 20:1). Thus, it appears that large, sterically hindering nitrogen protecting groups favor the production of the 4R,5S diastereomeric product, while less hindered blocking groups favor formation of the 4S,5S product. Aldehyde XVI was much less reactive than the t-Boc protected aldehyde [Chen, X., Hortelano, E. R., Eliel, E. L. J. Am. Chem. Soc., 112, 6130 (1990)]. Very little reaction occurred at -20 °C; the reaction went to completion over 24 h at 0 °C.

SCHEME IV

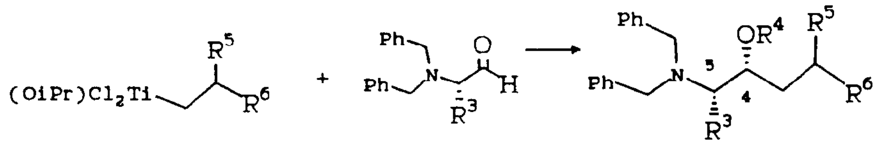

X XVI XVII

Reaction conditions detailed here do not cause any racemization of the sensitive aldehyde starting material. No 5R isomers were detected by chiral HPLC assay (Pirkle column) of hydroxyester or lactone products.

The compounds produced by the novel process of this invention are useful as intermediates in the preparation of peptides having defined stereochemistry. Furthermore, compounds having in vitro or in vivo renin or HIV protease inhibitory activity may be prepared directly according to the disclosed process.

Thus, in another preferred embodiment of this invention, a compound of structural formula XVIII:

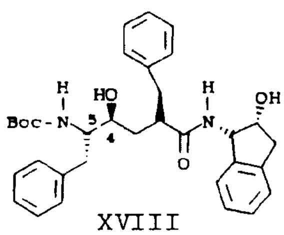

XVIII

useful as an inhibitor of the HIV protease, is prepared by reacting the aldehyde of structural formula XIX:

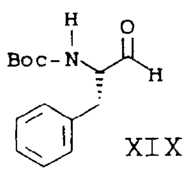

XIX

with a homoenolate of structural formula XX:

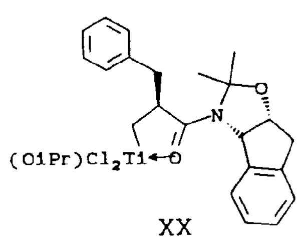

XX

followed by deprotection to the amide and hydroxyl containing compound XVIII (see Example 3). The reactant XX is prepared according to Scheme V and Example 3:

## SCHEME V

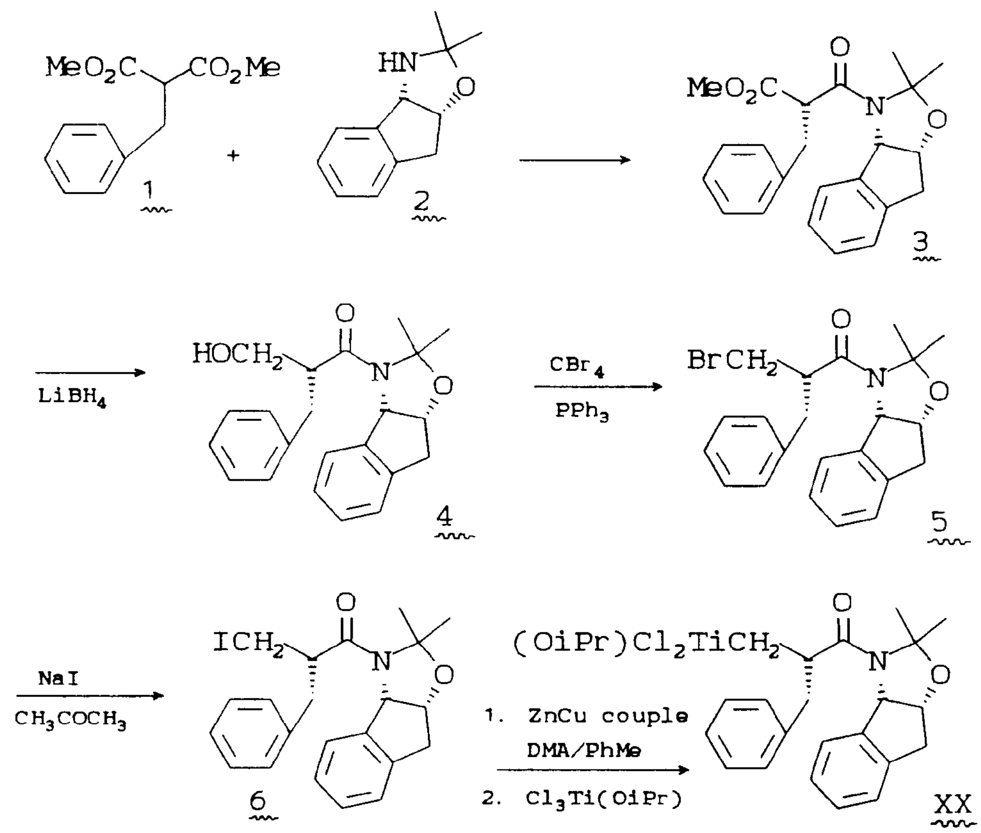


Compounds such as XVIII shown above may be prepared by the novel process of this invention in high yield with a high degree of stereochemical purity. Thus, compounds such as XVIII but having different $R^6$ groups, as defined above, may be prepared by this process. The process is useful to prepare diastereomeric compounds for in vitro testing as inhibitors of the HIV protease, renin, or other proteases of interest. Assays designed to test the potency of such compounds are known in the art. In the case of the HIV protease, a protease substrate, for example Val-Ser-Gln-Asn-Tyr-Pro-Ile-Val or Val-Ser-Gln-Asn-(betanapthyl)Ala-Pro-Ile-Val, is incubated with a preparation of the protease, (purified from virus, produced synthetically or produced by recombinant expression), in the presence or absence of inhibitory compounds. Inhibition of cleavage is monitored, for example, by HPLC. Thus, this process provides compounds useful as laboratory tools for studying proteolytic catalysis and its inhibition (see Example 4). XVIII in its deprotected form, (free amino and hydroxyl), is active in an assay designed to measure inhibition of the HIV protease.

Furthermore, HIV protease or renin inhibitory compounds produced by this process may be administered to patients in need of such treatment in pharmaceutical compositions comprising a pharmaceutical carrier and therapeutically-effective amounts of the compound or a pharmaceutically acceptable salt thereof. These pharmaceutical compositions may be in the form of orally-administerable suspensions or tablets; nasal sprays; sterile injectible preparations, for example, as sterile injectible aqueous or oleagenous suspensions or suppositories. The compositions may be comprised, in addition to the active HIV protease inhibitor and any inert carrier

substances, of anti-virals, immunomodulators, antibiotics, or vaccines thought to be useful in the treatment or prevention of HIV-disease (Marketletter, Nov. 30. 1987, 26-27; Genetic Engineering News, Jan. 1988, Vol. 8, 23]. Said compositions may comrise from 0.01 mg to 1 gram of the active compound and a like range of additional active or inert compounds.

Dosage levels on the order of 0.02 to 10 grams-per-day are useful in the inhibition of the HIV protease, the prevention or treatment of infection by HIV, or the treatment of AIDS or AIDS related complex (ARC). Preferred regimens for administration include doses of 10 to 50 milligrams per kilogram of body weight from one to three times per day, or in extreme cases as a constant intraveous infusion.

Representative experimental procedures utilizing the novel process are detailed below. These procedures are exemplary only and should not be construed as limiting on the novel process of this invention.

EXAMPLE 1

(5S)-5-[(1S)-1-(t-Boc-amino)-2-phenylethyl]-dihydro-furan-2(3H)-one:

The iodozinc-homoenolate was prepared by heating ethyl 3-iodopropionate (1.06 g, 4.66 mmol), freshly prepared zinc-copper couple (0.49 g, 7.53 mmol), anhydrous toluene (7.9 mL) and dimethylacetamide (0.79 mL) at ambient temperature for an hour followed by three hours at 80°C. The mixture was cooled to 22 °C and the excess zinc-coppper couple allowed to settle. To a flask equipped with a mechanical stirrer was added dry toluene (1.0 mL), dry $CH_2Cl_2$ (5.6 mL) and Ti(OiPr)4 (0.34 mL, 1.1 mmol). The solution was cooled to 17 °C and $TiCl_4$ (0.36 mL, 3.3 mmol) was added dropwise (< 25 °C). The pale yellow solution was stirred at room temp. for 15 min, and then cooled to -40 °C (a thin white slurry forms at the lower temperature). The iodozinc-homoenolate supernatant was added by cannula (leaving metal solids behind) to the $Cl_3Ti(OiPr)$ reaction mixture (<-20°C; a dark red solution formed). The resulting dichloroisopropoxy-titanium homoenolate solution was stirred at -25°C for 5 min. and then cooled to -40°C. A solution of t-Boc-(S)-phenylalaninal (0.55 g, 2.2 mmol) in $CH_2Cl_2$ (2 mL) was added by cannula to the homoenolate solution (< -35 °C). The reaction mixture was stirred vigorously at -20 °C for 18-24 h (TLC, 30 v/v% EtOAc in hexanes, cerric ammonium molybdate visualization). The reaction was quenched by addition to a stirred mixture of water (25 mL) and tert-butyl methyl ether (35 mL) at 0 °C (using dichloromethane to rinse the reaction flask of any thick residue). The mixture was stirred for 7 min (until a colorless two phase mixture was observed). The aqueous phase was extracted with tert-butyl methyl ether (25 mL), and the combined organic phases washed with 25 mL portions of water, saturated $NaHCO_3$ (two portions), water and brine. The organic phase was dried ($MgSO_4$) and evaporated in vacuo to give the hydroxyester (4S,5S predominating) as a white solid. The crude hydroxyester mixture was dissolved in toluene (18 mL) and glacial AcOH (0.55 mL) and heated at reflux for 2.5 h. The mixture was cooled to ambient temperature, diluted with isopropyl acetate, and washed with 25 mL portions of water, saturated $NaHCO_3$, water and brine. The organic phase was dried ($MgSO_4$), and evaporated in vacuo to give an oil which was crystallized from hexanes to give the title compound as a cream-colored solid (82% yield). The ratio of 4S,5S : 4R,5S diastereomers was determined by HPLC (Zorbax silica gel, normal phase column; mobile phase 90 :10 hexanes : isopropanol ;U.V. detection at 254 nm) to be 16 :1.

$^1$H NMR: (300 MHz, $CDCl_3$) δ 7.26 (m, 5H), 4.68 (d, 9.8 Hz, NH), 4.48 (td, J=7.7, 1.5 Hz, C4-H), 4.01 (m, C5-H), 2.82-3.02 (m, C6-H2), 2.51 (m, C2-H2), 2.12 (m, C3-H2), 1.37 (s, Me3C).

$^{13}$C NMR: δ 177.3 (Cl), 155.9 (N-C=O), 137.2 (ipso), 129.4, 128.7, 126.8, 80.0 (Me3C), 79.9 (C4), 54.1 (C5), 39.4 (C6), 28.8 (C2), 28.3 (Me3C), 24.2 (C3).

EXAMPLE 2

(5R)-5-[(1S)-1-(N,N-dibenzyamino)-2-phenylethyl]-dihydrofuran-2(3H)-one:

Essentially the same procedure as employed in Example 1 was used except that the t-Boc-(S)-phenylalaninal was replaced with N,N-dibenzyl analog to yield the title compound having a 4R,5S:4S,5S diastereomer ratio > 20:1.

$^1$H NMR: δ (300 MHz, $CDCl_3$) δ 7. 03- 7.43 (m, 15H), 4.71 (m, C4-H), 3.67 (s, N-$CH_2$-Ph), 2.91-3.15 (m, C6-H2, C5-H), 2.18-2.52 (overlapping m, C2-H2, C3-H), 1.75-1.91 (m, C3-H).

$^{13}$C NMR: δ 177.2 (Cl), 140.0 and 139.3 (ipso), 129.6, 128.7, 128.4, 128.2, 127.0 and 126.2 (para), 80.8 (C4), 62.5 (C5), 54.4 ($CH_2$-N), 32.5 , 28.2, 26.5.

EXAMPLE 3

N-(Cis-(-)-2-Hydroxy-1-Indanyl)-5(S)-(1,1-Dimethyl-ethoxycarbonylamino)-4(S)-Hydroxy-6-Phenyl-2(R)-Phenylmethylhexanamide, (See Scheme 5 above):

A solution of dimethyl-2-benzylmalonate 1 (2.1 g, 10 mmol) and protected indanol 2 (2.01 g, 10 mmol) in toluene (20 mL) is heated at reflux to yield 3. The mixture is cooled to room temperature, evaporated to dryness and chromatographed on silica gel to separate the diastereomers.

A solution of ester 3 (3.41 g, 0.9 mmol) in THF (50 mL) is cooled to 0°C. Lithium borohydride (0.39 g, 1.8 mmol) is added and the mixture stirred at 0°C-25°C until the reaction is complete. The mixture is poured into aqueous saturated ammonium chloride solution, extracted with ethylacetate (3 x 50 mL), dried over anhydrous $MgSO_4$, filtered and evaporated to give the crude product. The pure alcohol 4 is isolated by silica gel chromatography.

A solution of the alcohol 4 (2.81 g, 0.8 mmol, in dichloromethane (50 mL) is treated with triphenylphosphine (2.1 g, 0.8 mmol) and carbon tetrabromide (2.65 g, 0.8 mmol). The mixture is stirred at room temperature until the reaction is complete and then poured into water (50 mL). The organic phase is separated, washed with aqueous sodium bisulfite solution (50 mL, 10% solution), dried over anhydrous $MgSO_4$, filtered and evaporated to a crude residue which is purified by silica gel chromatography to give the bromide 5.

A solution of bromide 5 (2.89 g, 0.7 mmol) in acetone (50 mL) is treated with sodium iodide (1.05, 0.7 mmol) and stirred until the reaction is complete. The mixture is poured into water (50 mL) and extracted with diethylether (3 x 25 mL). The combined organic phases are washed with aqueous sodium bisulfite solution (50 mL, 10% aqueous solution) and saturated NaCl solution (50 mL). The organic phase is dried over anhydrous $MgSO_4$, filtered and evaporated to give the iodide 6. 6 is treated as in Scheme II with zinc copper couple followed by transmetallation with trichlorotitanium isopropoxide to yield XX. XX is then reacted with XIX to yield the protected form of XVIII as in Scheme III (wherein XIII is converted to XIV) and Example 1.

A solution of the protected XVIII amino alcohol (0.545 g, 1 mmol) in methanol (10 mL) is treated with camphor sulfonic acid (0.023 g, 0.1 mmol) at room temperature. The mixture is stirred at room temperature until the reaction is complete and then poured into diethylether (40 mL). The mixture is washed with aqueous sodium bicarbonate (25 mL) and water (25 mL). The organic phase is dried over anhydrous $MgSO_4$, filtered and evaporated to give the crude product which is purified by silica gel chromatography to yield the title compound, XVIII.

Example 4

HIV Protease Inhibition Assay:

Inhibition studies of the reaction of the protease expressed in Escherichia coli with a peptide substrate [Val-Ser-Gln-Asn-(betanapthyl)Ala-Pro-Ile-Val, 0.5 mg/mL at the time the reaction is inititated] were in 50 mM Na acetate, pH 5.5, at 30°C for 1 hour. Various concentrations of inhibitor in 1.0 μΛ DMSO were added to 25 μΛ of the peptide solution in water. The reaction is initiated by the addition of 15 μΛ of 0.33 nM protease (0.11 ng) in a solution of 0.133 M Na acetate pH 5.5 and 0.26% bovine serum albumin. The reaction was quenched with 160 μΛ of 5% phosphoric acid. Products of the reaction were separated by HPLC (VYDAC wide pore 5 cm C-18 reverse phase, acetonitrile gradient, 0.1% phosphoric acid). The extent of inhibition of the reaction was determined from the peak heights of the products. HPLC of the products, independently synthesized, proved quantitation standards and confirmation of the product composition.

## Claims

1. A process for making a compound of structural formula:

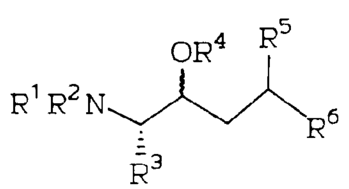

which comprises reacting about one equivalent of an N-protected-α-amino-aldehyde of structural formula:

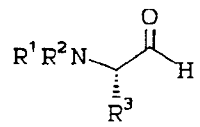

with between one and six equivalents of a homoenolate of structural formula:

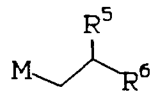

at a temperature between about -50°C and ambient temperature in a solvent selected from $CH_2Cl_2$, toluene, methyl-tert-butyl ether, diethyl ether, hexane, and $CCl_4$ or any combination of these, wherein:

$R^1$ and $R^2$ are independently:

a) hydrogen with the proviso that $R^1$, and $R^2$ may not both be H,
b) t-Boc, Aoc, Adc, Mcb, Mch, Cbz, Alloc, or Fmoc,
c) benzyl,
d) lower alkyl,
e) $CH_3O(CH_2CH_2O)_nCO$-, wherein n is from 1 to 10, or
f) tetrahydrofuryl-CO-;
g) R-CO- wherein R is an amino acid or peptide of up to five amino acids;

$R^3$ is:

a) benzyl,
b) cyclohexyl-$CH_2$-,
c) isobutyl,
d) benzyl-O-benzyl-, or
e) cinnamyl-;

$R^4$ is:

a) hydrogen,
b) a bond to $R^6$, when $R^6$ is carbonyl, or
c) (lower alkyl)$_3$Si-, t-butyl(lower alkyl)$_2$Si;

$R^5$ is:

a) hydrogen,
b) lower alkyl,
c) benzyl,
d) -$CH_2$-phenyl-O-$R^9$, or
e) -$CH_2$-CH=CH-phenyl-O-$R^9$;

$R^9$ is:

a) benzyl,
b)

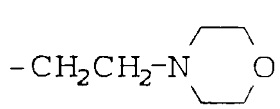
, or

c) -$CH_2CH_2$-O$R^{14}$;

$R^6$ is:

a) -COOR$^7$,
b) -CO-, when bonded to $R^4$ to form a lactone,

c)

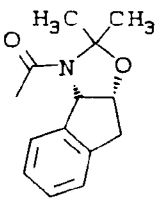

d)

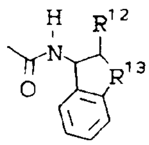

wherein:
$R^{12}$ is -H, or -OH;
$R^{13}$ is $-CH_2-O-$,

or 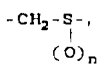

with n = 0, 1, or 2,
e)

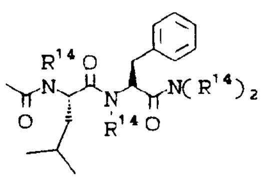

f)

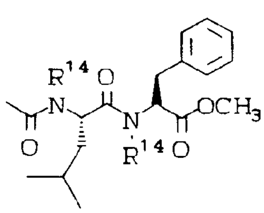

g)

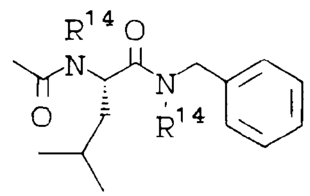

h)

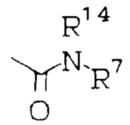

i)

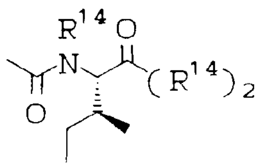

j)

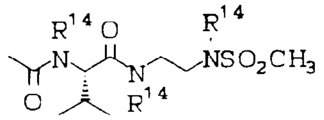

R7 is:

a) lower alkyl, or

b) benzyl;

$R^{14}$ is a removable protecting group selected from among:

a) t-Boc, Aoc, Adc, Mcb, Mch, Cbz, Alloc, or Fmoc, or

b) benzyl;

M is:

a) $TiR^8$,

b) $Sn(R^{10})_3$

c) $ZnR^{11}$,

d) $SmR^{11}$, or

e) $Ce(R^{11})_2$;

$R^8$ is:

a) $Cl_x(Oipr)_{3-x}$, or

b) $Br_x(Oipr)_{3-x}$;

x is an integer from 0 to 3;

$R^{10}$ is -lower alkyl, and

$R^{11}$ is I, Br, or Cl.

2. The process of Claim 1 wherein:
$R^1$ is hydrogen or benzyl;
$R^2$ is t-Boc, benzyl, or $CH_3O(CH_2CH_2O)_nCO$-, wherein n is from 1 to 10, or tetrahydrofuryl-CO-;
$R^4$ is hydrogen or a bond to $R^6$ if $R^6$ is carbonyl; and
M is $TiR^8$.

3. The process of Claim 2 wherein:
$R^1$ is hydrogen,
$R^2$ is t-Boc,
$R^3$ and $R^5$ are benzyl,
$R^4$ is hydrogen, and
$R^8$ is $Cl_2(OiPr)$.

4. The process of Claim 1 for making a compound of structural formula:

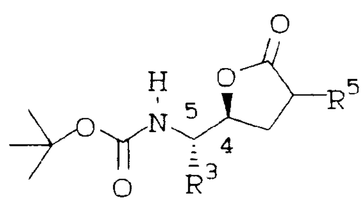

which comprises reacting an α-amino aldehyde of structural formula:

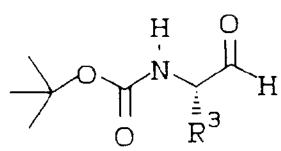

with a propionate homoenolate of structural formula:

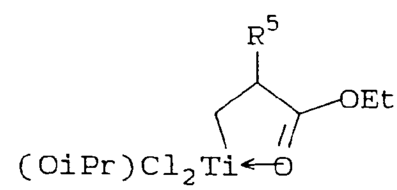

and then lactonizing the resulting hydroxyester of structural formula:

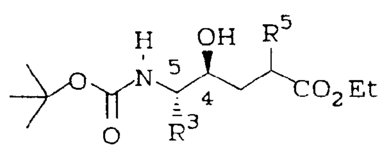

wherein:
$R^3$ is:
a) benzyl,
b) cyclohexyl-$CH_2$-,
c) isobutyl,
d) benzyl-O-benzyl-, or
e) cinnamyl;
$R^5$ is:

a) hydrogen,
b) lower alkyl,
c) benzyl,
d) $-CH_2$-phenyl-O-$R^9$, or
e) $-CH_2-CH=CH$-phenyl-O-$R^9$; and

$R^9$ is:
a) benzyl,
b)

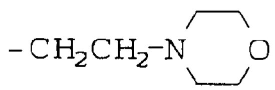

, or

c) $-CH_2CH_2-OR^{14}$.

**5.** The process of Claim 1 for making a compound of structural formula:

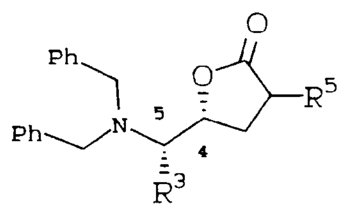

which comprises reacting an $\alpha$-amino aldehyde of structural formula:

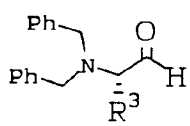

with a propionate homoenolate of structural formula:

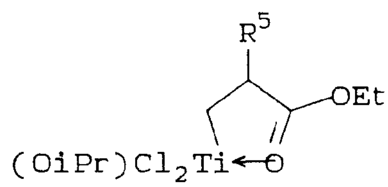

and then lactonizing the resulting hydroxyester of structural formula:

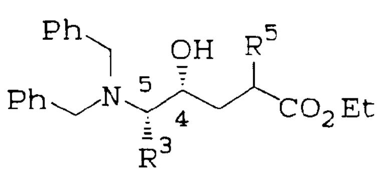

wherein:
$R^3$ is:
a) benzyl,
b) cyclohexyl-$CH_2$-,

c) isobutyl,
d) benzyl-O-benzyl-, or
e) cinnamyl;

$R^5$ is:
a) hydrogen,
b) lower alkyl,
c) benzyl,
d) $-CH_2$-phenyl-O-$R^9$, or
e) $-CH_2-CH=CH$-phenyl-O-$R^9$; and

$R^9$ is:
a) benzyl,
b)

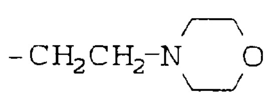

, or

c) $-CH_2CH_2-OR^{14}$.

**6.** The process of Claim 1 for making a compound of structural formula :

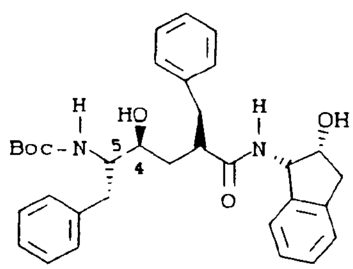

which comprises reacting an α-amino aldehyde of structural formula:

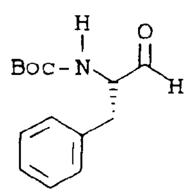

with a homoenolate of structural formula

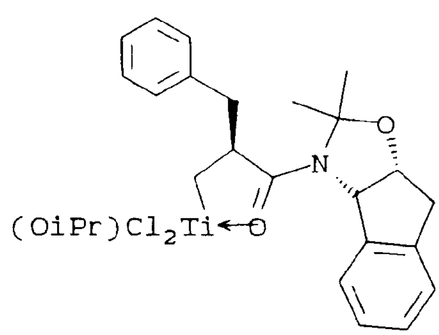

followed by deprotection to the free amino-alcohol.

European Patent Office

# EUROPEAN SEARCH REPORT

Application Number

EP 91 31 1659

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| D,A | EP-A-0 356 223 (MERCK)<br>* page 22, scheme I; examples 9, 10 *<br>--- | 1,4,6 | C07D307/33<br>C07D263/52<br>C07C237/22 |
| A | JOURNAL OF THE AMERICAN CHEMICAL SOCIETY, vol. 107, no. 7, 3 April 1985, Washington, DC, US, pages 2138 - 2141;<br>E. NAKAMURA ET AL.: 'Stereocontrolled construction of oxygenated steroidal side chains. Synthesis and stereochemistry of depresosterol'<br>* scheme I *<br>--- | 1 | |
| P,X | TETRAHEDRON LETTERS. vol. 32, no. 16, 15 April 1991, Oxford, GB, pages 1867 - 1870;<br>A.E. DECAMP ET AL.: 'Stereocontrolled addition of propionate equivalents to chiral alpha-amino aldehydes'<br>* the whole document *<br>--- | 1-5 | TECHNICAL FIELDS SEARCHED (Int. Cl.5 ) |
| P,X | TETRAHEDRON LETTERS. vol. 32, no. 2, 7 January 1991, Oxford, GB, pages 223 - 236;<br>S. KANO ET AL.: 'Stereocontrolled convergent synthesis of hydroxyethylene dipeptide isosteres by the reaction of alpha-amino aldehyde with alkoxytitanium homoenolates'<br>* the whole document *<br>----- | 1-5 | C07D<br>C07C |

The present search report has been drawn up for all claims

| Place of search | Date of completion of the search | Examiner |
|---|---|---|
| THE HAGUE | 30 MARCH 1992 | RUSSELL F. ENGLISH |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)